# EUROPEAN PATENT APPLICATION

(11) **EP 4 066 938 A1**
(43) Date of publication of application: **05.10.2022**
(21) Application number: 20893433.1
(22) Date of filing: 24.11.2020
(51) Int. Cl.: B01J 23/46, B01J 37/10, B01J 32/00, C07C 1/12, C07C 9/04, C10L 3/08

(54) **CATALYST FOR THE HYDROGENATION OF CO2 TO METHANE AT LOW TEMPERATURES**

(30) Priority: 26.11.2019 ES 201931042
(71) Applicant: Consejo Superior de Investigaciones Cientificas, 28010 Madrid (ES); Universitat Politècnica de València, 46022 Valencia (ES)
(72) Inventor: CORMA CANÓS, Avelino, 46022 Valencia (ES); HEYDORN, Patricia Concepción, 46022 Valencia (ES); CORED BANDRÉS, Jorge, 46022 Valencia (ES)
(74) Representative: Pons
(86) International application number: PCT/ES2020/070730
(87) International publication number: WO 2021/105537

(57) **Abstract**

The present invention describes a method for preparing a ruthenium catalyst with a carbon matrix, consisting of the following steps: (a) mixing a ruthenium compound with an organic compound in the presence of a solvent; (b) subjecting the mixture to hydrothermal conditions; and (c) washing and drying. The invention also relates to the catalyst obtained according to the method and to the use thereof in reactions to hydrogenate CO₂ to methane at low temperatures.

## Description

The present invention relates to a Ruthenium catalyst embedded in a carbon matrix with high catalytic efficiency at low reaction temperatures (< 200°C) in the hydrogenation of CO₂ to methane. The catalyst is prepared by a hydrothermal synthesis method at temperatures < 200°C. The present catalyst shows a methane space-time yield of 8.8 mol_{CH4} L⁻¹ h⁻¹ (3.2 µmol_{CH4} s⁻¹ g_{cat}⁻¹) at 160°C, operating at atmospheric pressure, 21428 h⁻¹ space velocity and H₂/CO₂ ratio of 3; and 160.8 Mol_{CH4}·L⁻¹·h⁻¹ (57.3 µmol_{CH4}·s⁻¹g_{cat}⁻¹) at 200°C, 20 bar, 21428 h⁻¹ and H₂/CO₂ = 3.

### BACKGROUND OF THE INVENTION

The use of CO₂ has garnered a great deal of attention as an alternative raw material to the use of fossil fuel sources for the synthesis of chemical products and fuels. Among the different catalytic processes, obtaining methane by hydrogenation of CO₂ is of particular interest, since it makes it possible to store the surplus of renewable energy in the form of CH₄, this surplus being easily transportable in current driving systems. Most of the processes for obtaining methane by hydrogenation of CO₂ are thermal processes that, unlike electro-catalytic and photo-catalytic processes, are much more efficient. The catalysts proposed in patents and in the literature for producing CH₄ from CO₂ are based on metals such as Ni, Ru, Pd, Rh, mono- or multimetallic, with or without promoters (Na, K, Cs, rare earth elements, etc.), metals supported on different metal oxides (TiO₂, SiO₂, Al₂O₃, CeO₂, ZrO₂) or carbon materials (N-doped CNT). For example, CN1 107078A describes a catalyst composed of Ni (2-40%), Ru (0.1-7 wt%) and sepiolite (60-98%), which operates at reaction temperatures of 400-450°C, space velocity of 6000-9000 h⁻¹, atmospheric pressure and H₂/CO₂ molar ratio of 4, which gives a methane space-time yield of 58 mol.L⁻¹.h⁻¹. Patent document JP01261202 describes a ruthenium catalyst on an alumina supporter, which operates at 300-400°C, space velocity of 670 h⁻¹ and pressure of 0.2 MPa to achieve 100% conversion of CO₂. In said document no results are given at reaction temperatures ≤ 200 °C. Methane production at a low temperature is reported by D.P. Becker et al. (Catal. Sci.Technol. 2016, 6, 8117-8128) using a Ru/TiO₂ catalyst synthesised by impregnation of a colloidal suspension of RuO₂ nanoparticles in TiO₂ (P25). 0.9 µmol_{CH4}.-s⁻¹.g_{cat}⁻¹ is obtained at 165°C and 2.57 µmol_{CH4}·s⁻¹·g_{cat}⁻¹ is obtained at 200°C with a contact time of 1.6 mL·s⁻¹g⁻¹ (F/W), H₂/CO₂ = 4 and atmospheric pressure. Patent document US4847231 shows the production of methane by irradiation with Xe light at temperatures of 25°C by using a mixture of Ru and RuOₓ supported on a photosensitive metal oxide. Photo-assisted CO₂ reduction produces CH₄ with an initial velocity of 116 µL h⁻¹ at 46°C by using a 20 cm³ reactor containing 1 mL of CO₂ and 12 mL of H₂ and illuminated with a 150-watt high pressure Xe lamp at an intensity of 0.08 W cm⁻². This result in 450 µL CH₄ in 5 h at 46°C. Patent document US3787468 describes the production of methane at a low temperature using a Ru metal catalyst doped on a WOₓ sheet (85:15 wt%) and using an electrochemical configuration that works as a catalytic reactor without electrical potential. The concentration of methane in the effluent gas is around 3000 ppm at 140°C using a Ru + WOₓ sheet with a metal load of 5 mg cm⁻², and a feed of 80% H₂, 20% CO₂ at 300 cm³ min⁻¹. In all these cases, the methane yield at low temperatures is low, where finding a catalyst with increased activity under mild reaction conditions is a significant development. This relates to reaction temperatures of < 200°C, atmospheric pressure and H₂/CO₂ ratios of ≤4. The catalysts reported so far show no activity under these conditions, high reaction temperatures (350-450°C), low space velocity (600 h⁻¹) and excess hydrogen (H₂/CO₂= 11-5) being required to increase the methane yield. This is energy-inefficient.

The present invention relates to a catalyst for the hydrogenation of CO₂ to methane with high activity at low temperatures (160-200°C). More particularly, it relates to a catalyst composed of ruthenium and carbon.

The present invention, compared to the state of the art, has the following advantages: 1) increased activity and selectivity to methane at low temperature (160-200°C); 2) low H₂/CO₂ ratio and high space velocity (21428 h⁻¹); 3) the method of preparation is easy and lacks aggressive reagents, water being the most suitable solvent.

### DESCRIPTION OF THE INVENTION

The present invention relates to a method for preparing a ruthenium catalyst with a carbon matrix, consisting of the following steps:
a) Mixing a ruthenium compound with an organic compound in the presence of a solvent.
b) Subjecting the mixture to hydrothermal conditions.
c) Washing and drying.

The method of the invention does not include a subsequent heat treatment step after step (c), where "heat treatment" is understood to be any process that includes calcination in any gas: inert gas (nitrogen, helium, argon), oxidizing gas (oxygen), reducing gas (hydrogen) or the reaction feed itself (CO₂:H₂ at molar ratios between 1-12) and which involves temperatures between 240-1000°C.

The absence of a subsequent heat treatment step has the following advantages:
- reduced cost associated with the gas consumption and energy consumption that heat treatment entails,
- reduced losses in mass of the obtained catalyst,
- easy implementation in industrial plants that have limits on the energy supply.

According to the present invention, the source of ruthenium is a Ru salt selected from nitrates, halides, sulphates, oxalates, acetates, citrates; Ru Complexes such as Ru(acac)₃, Ru-EDTA, Ru oxides, a metal-organic hybrid of the MOF type containing Ru such as, for example, Ru/UIO-66 composed of Zr clusters and 1,4-benzenedicarboxylate as a ligand, Ru/UIO-67 composed of Zr clusters and biphenyldicarboxylate as a ligand, and Ru-BTC (1,3,5-benzenetricarboxylate) and combinations thereof.

According to a particular embodiment, the organic compound used in the preparation is selected from a monosaccharide preferably selected from glucose, mannose, fructose, xylose and combinations thereof; a disaccharide preferably selected from sucrose, lactose, maltose and combinations thereof; a polysaccharide preferably selected from cellulose, glycogen, starch, chitin and combinations thereof; an organic compound containing at least two nitrogen atoms selected from ethylenediaminetetraacetic acid, the sodium salt of ethylenediaminetetraacetic acid, chitosan; and combinations thereof.

For the method described above, a solvent is required which is preferably selected from water or mixtures of water with alcohols having less than 11 carbon atoms, preferably between 2 and 8 carbon atoms in a ratio between 1:1 and 5:1 (v/v).

According to another particular embodiment, the ruthenium to carbon ratio on a molar basis of the catalyst prepared according to the method of the present invention is 0.003-0.4, preferably 0.005-0.1.

Furthermore, it is possible, in the method for obtaining the catalyst, for the latter to further comprise adding an additive. Preferably, said additive is nitrogen and can be in a molar ratio between 0.005-0.05 of the total catalyst

In the method of the present invention, the material is subjected to a hydrothermal method at a temperature that can be between 80 and 350°C, preferably between 120 and 250°C under static conditions for 1-30 h, preferably for 5-24 h.

Another aspect of the present invention relates to the ruthenium catalyst with a carbon matrix obtained according to the method described above, wherein said catalyst has a ruthenium to carbon ratio on a molar basis of 0.003-0.4, preferably 0.005-0.1.

In a more preferred embodiment of the catalyst of the present invention, said catalyst further comprises nitrogen in a molar ratio between 0.005-0.05 of the total catalyst.

The last aspect of the present invention relates to the use of the ruthenium catalyst with a carbon matrix described above and obtained according to the method described above, as a catalyst in hydrogenation reactions of CO₂ to methane at low temperatures, for example, in a continuous fixed bed reactor, preferably at temperatures between 80 and 450°C, more preferably between 110 and 300°C, and more preferably between 110 and 200°C. Said reactions can be carried out at a total pressure of between 1 and 80 bar, preferably between 1 and 40 bar, and more preferably between 1 and 30 bar. An excellent yield has been observed in the reaction for obtaining methane by hydrogenation of CO₂ operating under these conditions, which could lead to reduced energy consumption and the reduced cost of separating raw materials.

Moreover, according to a particular embodiment, the space velocity of the mentioned reactions, space velocity being understood as the number of volumes of catalytic bed fed per unit of time, is preferably between 5357 and 85714 h⁻¹, and more preferably between 6428 and 64284 h⁻¹. The molar ratio of H₂/CO₂ is between 1 and 12, preferably between 2 and 8.

The particle size of the catalyst can be between 400 and 600 µm, and can also be diluted in SiC (screened between 600 and 800 µm) in a weight ratio (catalyst: SiC) = 0.05-0.25, more preferably 0.14. According to a particular embodiment, the feed gas consists of (71-20) vol% H₂, (24-5) vol% CO₂ and (5-75) vol% N₂. The reaction products are analysed by gas chromatography. The space-time yield (STY) of methane in the present invention (see examples) is 8.8 mol_{CH4}L⁻¹ h⁻¹ (3.2 µmol s⁻¹ g_{cat}⁻¹) at 160°C, and 29.2 mol_{CH4}·L⁻¹·h⁻¹ (10.5 µmol·s⁻¹·g_{cat}⁻¹) at 200°C, operating at H₂/CO₂ = 3, 21428 h⁻¹ and 0.1 MPa. Operating at 2 MPa, the STY of CH₄ is 21.1 mol_{CH4}·L⁻¹·h⁻¹ (7.4 µmol·s⁻¹·g_{cat}⁻¹) at 160°C and 160.8 mol_{CH4}·L⁻¹·h⁻¹ (57.3 µmol·-s⁻¹g_{cat}⁻¹) at 200°C, operating at H₂/CO₂ = 3 and 21428 h⁻¹. These values are notably higher than those obtained in other conventional catalysts for obtaining methane by hydrogenation of CO₂. The selectivity to CH₄ is 99.9% in all cases.

Throughout the description and the claims, the word "comprises" and its variants do not intend to exclude other technical features, additives, components or steps. For those skilled in the art, other objects, advantages and features of the invention may be partially deduced from both the description and the embodiment of the invention.

### BRIEF DESCRIPTION OF THE FIGURES

**Figure 1** shows the conversion of CO₂ in Catalyst A at 160°C, 0.1 MPa, 21428 h⁻¹ space velocity and the feed composition of 5 vol% CO₂, 20 vol% H₂, 75 vol% N₂
**Figure 2** shows the conversion of CO₂ in Catalyst B at 160°C, 0.1 MPa, 21428 h⁻¹ space velocity and the feed composition of 5 vol% CO₂, 20 vol% H₂, 75 vol% N₂
**Figure 3** shows the conversion of CO₂ in Catalyst A at 200°C, 2 MPa, 21428 h⁻¹ space velocity and the feed composition of 23.8 vol% CO₂, 71.3 vol% H₂, 5 vol% N₂

The present invention is illustrated by means of the following examples not intended to be limiting thereof.

### EXAMPLES

### Example 1: Synthesis of Catalyst A

120 mg of glucose (Aldrich) dissolved in 7 mL of deionised water are stirred at room temperature for 0.5 h. Next, 0.1 g of RuO₂ (Aldrich, 99.9%, particle size 32 nm determined by XRD) is added and mixed under ultrasound (Branson 3510 operating at 40 Hz) for 0.5 h, obtaining a black suspension. The suspension thus obtained is transferred to a 12.5 mL Teflon-lined stainless-steel autoclave. The autoclave is closed, introduced in an oven set at 175°C and maintained for 18 h under static conditions. After removing the autoclave from the oven, it is cooled to room temperature for 2 h. The contents of the autoclave are filtered under vacuum, recovering a black solid. The solid is washed five times first with water and then with acetone. It is then dried in an oven at 60°C for 12 h. A final amount of 91 mg of solid product is recovered. The catalyst thus obtained is called Catalyst A, with 0.2 mol% Ru, according to an inductively coupled plasma (ICP) analysis. Nitrogen is not present in the sample based on elemental analysis (AAS) and X-ray photoelectron spectroscopy (XPS) data.

### Example 2: Synthesis of Catalyst B

5.26 g Ru(acac)₃, 1.77 g Na₂EDTA and 0.385 g NaOH are dissolved in 8.2 mL of deionised water. Next, 4 mLmethanol are added to the aqueous solution under stirring at room temperature obtaining a red suspension, which is transferred to a 35 mL Teflon-lined stainless steel autoclave followed by static hydrothermal processing at 200°C for 24 h. Next, the autoclave is removed from the oven and cooled to room temperature for 3 h. After cooling to room temperature, the precipitate generated is filtered and washed five times with deionised water and acetone. The catalyst thus obtained is called Catalyst B, with 0.2 mol% Ru according to the ICP analysis. Nitrogen is present at 0.005 mol%.

### Example 3: Catalytic behaviour at 0.1 MPa

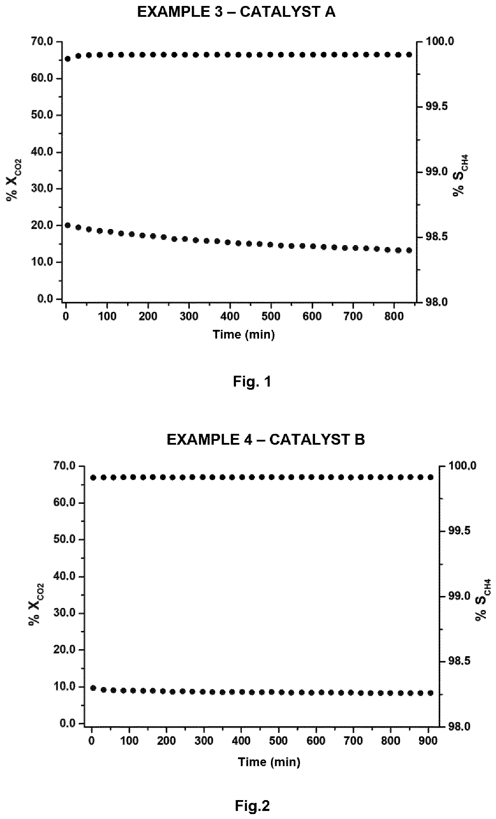

The catalytic behaviour of Catalyst A is tested in a flow fixed bed reactor. 206 mg of catalyst are diluted with 1500 mg of SiC and packed into a stainless-steel reaction tube with an 11 mm inner diameter and a length of 240 mm. The volume of the catalytic bed is 0.28 cm³. The catalyst is activated in a stream of 23.8 vol% CO₂, 71.3 vol% H₂, 5 vol% N₂, 100 ml min⁻¹ total flow at atmospheric pressure for 2 h at 160°C. Next, the reaction mixture is changed to 5 mL min⁻¹ CO₂, 20 mL min⁻¹ H₂ and 75 mL min⁻¹ N₂. The reaction products are analysed by GC using a MS-13X column and a TCD detector. Multiple analyses, each with a duration of 22 minutes, are acquired for a total of 14.5 h of reaction time. **Figure 1** shows the time evolution in the conversion of CO₂ in Catalyst A at 160°C. The selectivity to methane is 99.9%.

The data in **Figure 1** corresponds to 13.2% CO₂ conversion, and a methane yield of 6.3 mol_{CH4} L⁻¹ h⁻¹ (2.4 µmol s⁻¹ g_{cat}⁻¹).

### Example 4: Catalytic behaviour at 0.1 MPa

The catalytic behaviour of Catalysts A and B is tested in the same reactor configuration as in Example 3. The pre-activation of Catalyst B takes place in 5 mL min⁻¹ CO₂, 20 mL min⁻¹ H₂ and 75 mL min⁻¹ N₂. The gas mixture is fed at 25°C and subsequently, the temperature is raised to 160°C (10°C min⁻¹, 2.5 h) and 180°C (10°C min⁻¹, 7.5 h). The pre-activation of Catalyst A takes place in 23.8 mL min⁻¹ CO₂, 71.3 mL min⁻¹ H₂ and 5 mL min⁻¹ N₂ at 160°C for 2 h.

After activation, the catalytic studies are carried out where the reaction mixture in both cases is formed by 5 mL min⁻¹ CO₂, 20 mL min⁻¹ H₂ and 75 mL min⁻¹ N₂. The reaction temperatures are 160°C, 180°C and 200°C with a temperature rise ramp of 10°C min⁻¹. **Table 1** shows the conversion of CO₂ (X_{CO2}, %), methane yield (STY_{CH4}) (mol L⁻¹ h⁻¹ and µmol s⁻¹ g_{cat}⁻¹) in Catalysts A and B. **Figure 2** shows the conversion of CO₂ at 160°C in Catalyst B.

**TABLE 1**

| | **CATALYST A** | | | | **CATALYST B** | | | |
|---|---|---|---|---|---|---|---|---|
| **T (°C)** | **TOS (h)** | **X_{CO2} (%)** | **Y_{CH4} (µmol·s⁻¹·gca⁻¹)** | **STY_{CH4} (mol·L⁻¹·h⁻¹)** | **TOS (h)** | **X_{CO2} (%)** | **Y_{CH4} (µmol**·**s⁻¹g_{cat}⁻¹)** | **STY_{CH4} (mol·L⁻¹·h⁻¹)** |
| 160 | 14.5 | 13.2 | 2.4 | 6.3 | 15.5 | 8.4 | 1.5 | 4.0 |

| **T (°C)** | **TOS (h)** | **X_{CO2} (%)** | **Y_{CH4}** (**µmol·s⁻¹·g_{cat}⁻¹)** | **STY_{CH4} (mol·L⁻¹·h⁻¹)** | **TOS (h)** | **X_{CO2} (%)** | **Y_{CH4}** (**µmol·s⁻¹·g_{cat}⁻¹)** | **STY_{CH4} (mol·L⁻¹·h⁻¹)** |
|---|---|---|---|---|---|---|---|---|
| 180 | 7.0 | 29.9 | 5.4 | 14.3 | 2.3 | 28.5 | 5.0 | 13.6 |
| 200 | - | - | - | - | 19.4 | 51.4 | 9.1 | 24.6 |

The data in **Figure 2** corresponds to 8.4% CO₂ conversion and methane yield of 4.0 mol L⁻¹ h⁻¹ (1.5 µmol s⁻¹.g_{cat}⁻¹).

### Example 5: Catalytic behaviour at 0.1 MPa

The catalytic behaviour of Catalyst B is tested in the same reactor configuration as in example 3. Catalyst B is brought into contact with 23.8 mL min⁻¹ CO₂, 71.3 mL min⁻¹ H₂ and 5 mL min⁻¹ N₂, at 35°C and 0.1 MPa. After that, the temperature is increased by 10°C min⁻¹ at 160°C, 180°C and 200°C. The catalyst is maintained for 2 h at each temperature. **Table 2** shows the conversion of CO₂ (X_{CO2}, %) and methane yield (STY_{CH4}) (mol L⁻¹ h⁻¹ and µmol s⁻¹ g_{cat}⁻¹) in Catalyst B at each reaction temperature.

**TABLE 2**

| **CATALYST B** | | | | |
|---|---|---|---|---|
| **T (°C)** | **TOS (h)** | **X_{CO2} (%)** | **Y_{CH4}** (**µmol·s⁻¹·g_{cat}⁻¹)** | **STY_{CH4} (mol·L⁻¹·h⁻¹)** |
| 160 | 1.2 | 3.9 | 3.2 | 8.8 |
| 180 | 2.5 | 6.6 | 5.5 | 14.9 |
| 200 | 1.5 | 12.7 | 10.5 | 29.2 |

### Example 6: Catalytic behaviour at 2 MPa

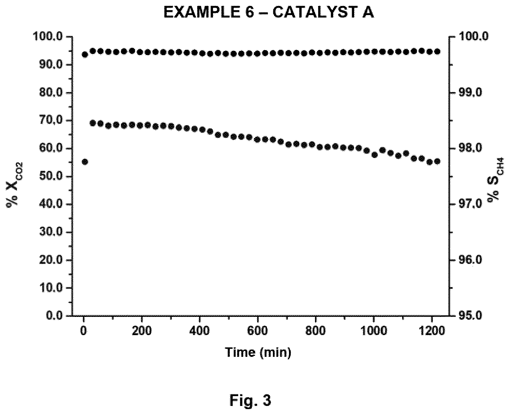

The catalytic behaviour of Catalyst B is tested in the same reactor configuration as in example 3. The catalyst is initially activated in 20.0 mL min⁻¹ H₂ and 80.0 mL min⁻¹ N₂ at atmospheric pressure (16.5 h at 160°C and 6.5 h at 180°C), and then in 71.3 mL min⁻¹ H₂ and 28.7 mL min⁻¹ N₂ at 2 MPa (1.5 h at 160°C, 1.5 h at 180°C and 3.5 h at 200°C). After activation, at 200°C the feed is changed to 23.8 mL min⁻¹ CO₂, 71.3 mL min⁻¹ H₂ and 5 mL min⁻¹ N₂ at 2 MPa. Under these conditions, a conversion of 70% CO₂, with a methane yield of 160.8 mol L⁻¹ h⁻¹ (57.3 µmol s⁻¹ g_{cat}⁻¹), is obtained, **Figure 3****.**

The same catalytic behaviour is achieved when the catalyst is activated in 23.8 mL min⁻¹ CO₂, 71.3 mL min⁻¹ H₂ and 5 mL min⁻¹ N₂ at 2 MPa for 2.5 h at 160°C and 3 h at 180°C.

### Example 7: Synthesis of a reference catalyst (C)

0.396 g of Ru(acac)₃ are dissolved in 20 mL of toluene for 0.5 h. Next, 0.9 g of carbon (Norit activated carbon) are added and kept stirring for 15 h. The final solution is evaporated under vacuum resulting in a black solid. The solid is reduced in 50 mL min⁻¹ H₂ at 250°C for 3 h with a heating ramp of 10°C min⁻¹, followed by cooling in N₂ at 25°C. It is then calcined in 50 ml min⁻¹ flow of O₂ at 400°C for 3 h. The catalyst thus obtained is called Catalyst C, with 0.04 mol% Ru according to the ICP analysis.

### Example 8: Catalytic behaviour of the reference catalyst

The catalytic behaviour of Catalyst C is tested in the same configured reactor as in example 3. Catalyst C is directly brought into contact with 13.8 mL min⁻¹ CO₂, 71.3 mL min⁻¹ H₂ and 5 mL min⁻¹ N₂, at 160°C and 0.1 MPa for 1.5 h. Then the pressure is increased to 2 MPa, and the temperature to 160°C and 200°C. The catalyst remains for about 1.5 h at each temperature. **Table 3** shows the conversion of CO₂ (X_{CO2}, %) and the methane yield (STY_{CH4}) (mol L⁻¹ h⁻¹ and µmol s⁻¹ g_{cat}⁻¹) in Catalyst C.

**TABLE 3**

| **CATALYST C (Reference)** | | | | | |
|---|---|---|---|---|---|
| **T (°C)** | **TOS (h)** | **P (MPa)** | **X_{CO2} (%)** | **Y_{CH4} (µmol·s⁻¹·g_{cat}⁻¹)** | **STY_{CH4} (mol·L⁻¹·h⁻¹)** |
| 160 | 1.5 | 0.1 | <0.1 | <0.1 | <0.1 |
| 160 | 1.5 | 2 | 0.1 | <0.1 | 0.1 |
| 200 | 1.5 | 2 | 0.2 | 0.1 | 0.2 |

### Example 9: Catalytic behaviour of Catalyst A calcined in an inert gas at 600°C

Catalyst A is calcined in an inert gas at 600°C in a Helium stream (15 ml min⁻¹ at 10°C min⁻¹ for 2 h). The catalyst thus obtained is called Catalyst D. The catalytic behaviour of Catalyst D is tested in the same reactor configuration as in example 3. Catalyst D is brought into contact with 23.8 mL min⁻¹ CO₂, 71.3 mL min⁻¹ and 5.0 mL min⁻¹ N₂ at 25°C and 0.1 MPa. After that, the temperature is increased by 10°C min⁻¹ to 160°C and 180°C. The catalyst is maintained for 2.5 h at each temperature. **Table 4** shows the conversion of CO₂ (X_{CO2}, %) and methane yield (STY_{CH4}) (mol L⁻¹ h⁻¹ and µmol s⁻¹ g_{cat}⁻¹) of Catalyst D at each reaction temperature compared to its reference (Catalyst A).

**TABLE 4**

| | **CATALYST A** | | | | **CATALYST D** | | | |
|---|---|---|---|---|---|---|---|---|
| **T (°C)** | **TOS (h)** | **X_{CO2} (%)** | **Y_{CH4}** (**µmol·s⁻¹·g_{cat}⁻¹)** | **STY_{CH4} (mol·L⁻¹·h⁻¹)** | **TOS (h)** | **X_{CO2} (%)** | **Y_{CH4}** (**µmol·s⁻¹·g_{cat}⁻¹)** | **STY_{CH4} (mol·L⁻¹·h⁻¹)** |
| 160 | 2.5 | 3.8 | 3.2 | 8.8 | 2.5 | 0.7 | 0.7 | 1.8 |
| 180 | 2.5 | 5.8 | 5.1 | 14.1 | 2.5 | 2.1 | 2.0 | 5.6 |

It is clearly seen how reference Catalyst A (not calcined) has a much better conversion to CO₂ and methane yield than Catalyst D (calcined).

### Example 10: Catalytic behaviour of the reduced Catalyst D in H₂ at 250°C for 1 h.

Catalyst D is activated in 25 mL min⁻¹ H₂ at atmospheric pressure (1.0 h at 250°C, 10°C min⁻¹). The catalytic behaviour of Catalyst D is tested in the same reactor configuration as in example 3. After activation, the temperature is lowered to 160°C by changing the feed to 23.8 mL min⁻¹ CO₂, 71.3 mL min⁻¹ and 5.0 mL min⁻¹ N₂ (0.1 MPa). Subsequently, the temperature is increased to 180°C. The catalyst is maintained for 2.5 h at each temperature.

### Table 5 shows the conversion of CO₂ (X_{CO2}, %) and methane yield (STY_{CH4}) (mol L⁻¹ h⁻¹ and µmol s⁻¹ g_{cat}⁻¹) of reduced Catalyst D at each reaction temperature compared to its reference (Catalyst A).

**TABLE 5**

| | **CATALYST A** | | | | **CATALYST D-reduced** | | | |
|---|---|---|---|---|---|---|---|---|
| **T (°C)** | **TOS (h)** | **X_{CO2} (%)** | **Y_{CH4}** (**µmol·s⁻¹·g_{cat}⁻¹)** | **STY_{CH4} (mol·L⁻¹·h⁻¹)** | **TOS (h)** | **X_{CO2} (%)** | **Y_{CH4}** (**µmol·s⁻¹·g_{cat}⁻¹)** | **STY_{CH4} (mol·L⁻¹·h⁻¹)** |
| 160 | 2.5 | 3.8 | 3.2 | 8.8 | 2.5 | 0.6 | 0.5 | 1.4 |
| 180 | 2.5 | 5.8 | 5.1 | 14.1 | 2.5 | 1.7 | 1.6 | 4.4 |

It is clearly seen how reference Catalyst A (not calcined) has a much better conversion to CO₂ and methane yield than reduced Catalyst D (calcined).

## Claims

1. A method for preparing a ruthenium catalyst with a carbon matrix, **characterised in that** it consists of the following steps:
a) Mixing a ruthenium compound with an organic compound in the presence of a solvent.
b) Subjecting the mixture to hydrothermal conditions.
c) Washing and drying.

2. The method for preparing a catalyst according to claim 1, **characterised in that** the source of ruthenium is a Ru salt selected from nitrates, halides, sulphates, oxalates, acetates, citrates; Ru Complexes such as Ru(acac)₃, Ru-EDTA, Ru oxide, a metal-organic hybrid of the MOF type containing Ru and combinations thereof.

3. The method for preparing a catalyst according to one of claims 1 and 2, **characterised in that** the organic compound is selected from
• a monosaccharide,
• a disaccharide,
• a polysaccharide
• an organic compound containing at least two nitrogen atoms selected from ethylenediaminetetraacetic acid, the sodium salt of ethylenediaminetetraacetic acid, chitosan and combinations thereof,
• and combinations thereof.

4. The method for preparing a catalyst according to claim 3, **characterised in that** the organic compound is a monosaccharide selected from glucose, mannose, fructose, xylose and combinations thereof.

5. The method for preparing a catalyst according to claim 3, **characterised in that** the organic compound is a disaccharide selected from sucrose, lactose, maltose and combinations thereof.

6. The method for preparing a catalyst according to claim 3, **characterised in that** the organic compound is a polysaccharide selected from cellulose, glycogen, starch, chitin and combinations thereof.

7. The method for preparing a catalyst according to claim 3, **characterised in that** the organic compound contains at least two nitrogen atoms and is selected from ethylenediaminetetraacetic acid, the sodium salt of ethylenediaminetetraacetic acid, chitosan, and combinations thereof.

8. The method for preparing a catalyst according to one of the preceding claims, **characterised in that** the solvent is selected from water or mixtures of water with alcohols having less than 11 carbon atoms in a ratio between 1:1 and 5: 1 (v/v).

9. The method for preparing a catalyst according to one of the preceding claims, **characterised in that** the method for preparing the catalyst follows a hydrothermal method at a temperature between 80 and 350°C under static conditions for 1-30 h.

10. The method for preparing a catalyst according to claim 9, **characterised in that** the temperature at which the hydrothermal method is carried out is between 120-250°C, under static conditions for 5-24 h.

11. A ruthenium catalyst with a carbon matrix obtained according to any of claims 1 to 10, **characterised in that** it has a ruthenium to carbon ratio on a molar basis of 0.003-0.4.

12. The catalyst according to claim 11, **characterised in that** the ruthenium to carbon ratio on a molar basis is 0.005-0.1.

13. The catalyst according to any of claims 11 or 12, **characterised in that** it comprises nitrogen in a molar ratio between 0.005-0.05 of the total catalyst.

14. Use of the ruthenium catalyst with a carbon matrix described in any of claims 11 to 13 and obtained according to the method of preparation of claims 1 to 10, as a catalyst in hydrogenation reactions of CO₂ to methane at temperatures between 80 and 450°C.

15. The use of the ruthenium catalyst with a carbon matrix according to claim 14, **characterised in that** the temperature of the hydrogenation reaction of CO₂ to methane is between 110 and 300°C.

16. The use of the ruthenium catalyst with a carbon matrix according to any of claims 14 or 15, **characterised in that** it is carried out at a total pressure of between 1 and 80 bar.

17. The use of the ruthenium catalyst with a carbon matrix according to claim 16, **characterised in that** the process is carried out at a pressure between 1 and 40 bar.

18. The use of the ruthenium catalyst with a carbon matrix according to one of claims 14 to 17, **characterised by** a space velocity between 5357 and 85714 h⁻¹.

19. The use of the ruthenium catalyst with a carbon matrix according to claim 18, **characterised by** a space velocity between 6428 and 64284 h⁻¹.

20. The use of the ruthenium catalyst with a carbon matrix according to one of claims 14 to 19, **characterised in that** the molar ratio of H₂/CO₂ is between 1 and 12.

21. The use of the ruthenium catalyst with a carbon matrix according to claim 20, **characterised in that** the molar ratio of H₂/CO₂ is between 2 and 8.
